# EUROPEAN PATENT APPLICATION

(11) **EP 4 729 045 A1**
(43) Date of publication of application: **22.04.2026**
(21) Application number: 24825863.4
(22) Date of filing: 17.06.2024
(51) Int. Cl.: A61K 8/68, A61K 8/06, A61Q 1/14, A61Q 19/00

(54) **OIL-IN-WATER EMULSION COMPOSITION**

(30) Priority: 19.06.2023 JP 2023100122
(71) Applicant: Kao Corporation, Tokyo 103-8210 (JP)
(72) Inventor: TABUCHI, Sho, Tokyo 103-8210 (JP); HIRATA, Tomohiro, Tokyo 103-8210 (JP); KINOSHITA, Takafumi, Tokyo 103-8210 (JP); OKUBO, Minami, Tokyo 103-8210 (JP); IKOSHI, Risa, Tokyo 103-8210 (JP)
(74) Representative: Hoffmann Eitle
(86) International application number: PCT/JP2024/021901
(87) International publication number: WO 2024/262462

(57) **Abstract**

An oil-in-water emulsion composition including the following components (A), (B), (C), and (D): (A) a ceramide, (B) an oil agent in a liquid state at 25°C, (C) a nonionic surfactant having HLB of less than 10, and (D) water, wherein a Z-average particle size of emulsion particles is from 0.05 to 0.3 um.

## Description

### Field of the Invention

The present invention relates to an oil-in-water emulsion composition and a method for producing the same.

### Background of the Invention

In the spaces among corneocytes constituting the stratum corneum located in the outermost layer of the skin, lipid which is called stratum corneum intercellular lipid is present. Ceramides accounting for approximately 50% of this stratum corneum intercellular lipid are deeply involved in rough skin and dry skin. It is known that the state of the stratum corneum can be improved by supplementation with ceramides by external use.

However, for formulating solid fat such as ceramides into cosmetics, the solid fat is difficult to stabilize in formulations due to its high crystallinity and high melting point. Therefore, techniques of stably formulating such solid fat are under investigation.

For example, Patent Literature 1 recites that a translucent cosmetic containing a ceramide, an oil component, a nonionic surfactant, and water, wherein an average particle size of an O/W emulsion constituted thereby is from 100 nm to 300 nm is excellent in time-dependent stability and has beautiful appearance.

(Patent Literature 1) WO 2004/045566

### Summary of the Invention

The present invention relates to an oil-in-water emulsion composition comprising the following components (A), (B), (C), and (D):
(A) a ceramide,
(B) an oil agent in a liquid state at 25°C,
(C) a nonionic surfactant having HLB of less than 10, and
(D) water, wherein
   a Z-average particle size of emulsion particles is from 0.05 to 0.3 µm.

The present invention also relates to a method for producing the oil-in-water emulsion composition, comprising: a step 1 of obtaining an oil phase containing the component (A), the component (B), and the component (C); and a step 2 of mixing the oil phase obtained in the step 1 with an aqueous phase containing the component (D).

### Detailed Description of the Invention

Conventional cosmetics may not sufficiently impart smoothness or moist feeling in the skin after application. Another problem thereof is preservation stability.

The present inventors found that a ceramide, a liquid oil agent, and a specific nonionic surfactant are used in combination, whereby an oil phase containing a ceramide is stably emulsified and dispersed as fine particles to produce an oil-in-water emulsion composition which is excellent in preservation stability and imparts smoothness and excellent moist feeling in the skin after application.

The oil-in-water emulsion composition of the present invention is excellent in preservation stability and can impart smoothness and excellent moist feeling in the skin after application because an oil phase containing a ceramide is stably emulsified and dispersed as fine particles.

Examples of the ceramide as the component (A) used in the present invention may include natural ceramide such as ceramides 1, 2, 3, 4, 5, 6, and 7, and sphingosine derivatives such as phytosphingosine as well as ceramide analog structural substances recited in JP-AS62-228048, JP-A-S63-216812, JP-A-S63-227513, JP-A-S64-29347, JP-A-S64-31752, and JP-A-H8-319263. Examples of the ceramide analog structural substance specifically include compounds of the following formulas (1) and (2) from the viewpoint of creating fine particles of an oil containing a ceramide and from the viewpoint of improving preservation stability.

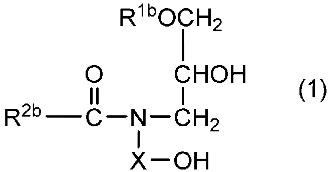

In the formula, R^{1b} represents a hydrocarbon group having 10 to 26 carbon atoms, R^{2b} represents a hydrocarbon group having 9 to 25 carbon atoms, and X represents -(CH₂)ₙ- (wherein n represents an integer of from 2 to 6).

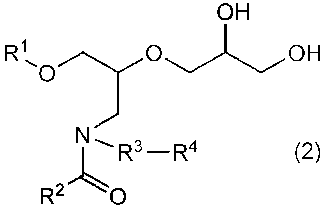

In the formula, R¹ and R² are the same or different and each represent an optionally hydroxylated hydrocarbon group having 1 to 40 carbon atoms, R³ represents an alkylene group having 1 to 6 carbon atoms or a single bond, and R⁴ represents a hydrogen atom, an alkoxy group having 1 to 12 carbon atoms, or 2,3-dihydroxypropyloxy group, on the proviso that when R³ is a single bond, R⁴ is a hydrogen atom.

In the formulas (1) and (2), the hydrocarbon group is preferably an alkyl group or an alkenyl group.

Examples of the compound of the formula (1) may include N-(hexadecyloxyhydroxypropyl)-N-hydroxyethylhexadecanamide. Examples of the compound of the formula (2) may include long-chain dibasic acid bis-3-methoxypropylamide.

The compound of the formula (1), a pseudoceramide, is a ceramide functional component and can improve skin conditions (moist content, and the like) by supplementing the action of ceramides.

The component (A) preferably includes one or more members selected from the group consisting of natural ceramide, sphingosines, and compounds of the formulas (1) and (2), more preferably one or more members selected from the group consisting of natural ceramide, sphingosines, and a compound of the formula (1), further more preferably one or more members selected from the group consisting of natural ceramide, phytosphingosine, and N-(hexadecyloxyhydroxypropyl)-N-hydroxyethylhexadecanamide, and even more preferably N-(hexadecyloxyhydroxypropyl)-N-hydroxyethylhexadecanamide, from the viewpoint of creating fine particles of an oil containing a ceramide and from the viewpoint of improving preservation stability.

One or more members can be used as the component (A). The content thereof is preferably 0.3% by mass or more, more preferably 0.5% by mass or more, further more preferably 0.6% by mass or more, even more preferably 0.7% by mass or more, further more preferably 0.8% by mass or more, and preferably 4% by mass or less, more preferably 3% by mass or less, further more preferably 2% by mass or less, even more preferably 1.8% by mass or less, further more preferably 1.3% by mass or less, in the whole composition from the viewpoint of creating fine particles of an oil containing a ceramide, from the viewpoint of improving preservation stability, and from the viewpoint of improving moist feeling in the skin after application.

The content of the component (A) is preferably from 0.3 to 4% by mass, more preferably from 0.5 to 3% by mass, further more preferably from 0.6 to 2% by mass, even more preferably from 0.7 to 1.8% by mass, further more preferably from 0.8 to 1.3% by mass, in the whole composition.

The content of the component (A) is preferably from 0.3 to 2% by mass, more preferably from 0.3 to 1.8% by mass, further more preferably from 0.5 to 1.5% by mass, in the whole composition.

The component (B) is an oil agent in a liquid state at 25°C. The liquid state refers to having fluidity and also includes a paste state.

Examples of the oil agent as the component (B) may include hydrocarbon oils, ester oils, ether oils, higher alcohol, silicone oils, and higher fatty acids.

More specifically, examples of the hydrocarbon oil may include squalane, liquid paraffin, liquid isoparaffin, light liquid isoparaffin, and heavy liquid isoparaffin.

The hydrocarbon oil preferably includes one or more members selected from the group consisting of squalane, liquid paraffin, liquid isoparaffin, and light liquid isoparaffin, more preferably one or more members selected from the group consisting of squalane, liquid paraffin, and liquid isoparaffin, further more preferably one or more members selected from the group consisting of squalane and liquid isoparaffin, and even more preferably liquid isoparaffin, from the viewpoint of creating fine particles of an oil containing a ceramide, from the viewpoint of improving preservation stability, from the viewpoint of improving smoothness in the skin after application, and from the viewpoint of improving moist feeling in the skin after application. The liquid isoparaffin is also called hydrogenated polyisobutene.

Examples of the ester oil may include monoester oils, diester oils, triester oils, and tetraester oils.

Examples of the monoester oil may include monoester of aliphatic or aromatic monocarboxylic acid or dicarboxylic acid having 2 to 24 carbon atoms. Specific examples thereof include cetyl 2-ethylhexanoate, cetyl octanoate, isononyl isononanoate, isotridecyl isononanoate, isostearyl isostearate, hexyl laurate, isopropyl myristate, octyldodecyl myristate, myristyl myristate, 2-hexyldecyl myristate, isopropyl palmitate, octyl palmitate, 2-hexyldecyl palmitate butyl stearate, isocetyl stearate, isocetyl isostearate, decyl oleate, isodecyl benzoate, octyl methoxycinnamate, hexyldecyl dimethyloctanoate, cetyl lactate, myristyl lactate, lanoline acetate, 2-ethylhexyl succinate, 2-hexyldecyl adipate, alkyl (C12 to C15) benzoate, and jojoba oil.

The monoester oil preferably includes one or more members selected from the group consisting of isononyl isononanoate, isotridecyl isononanoate, isostearyl isostearate, octyldodecyl myristate, and jojoba oil, more preferably one or more members selected from the group consisting of isononyl isononanoate, isotridecyl isononanoate, isostearyl isostearate, and jojoba oil, and further more preferably isononyl isononanoate, from the viewpoint of creating fine particles of an oil containing a ceramide, from the viewpoint of improving preservation stability, from the viewpoint of improving smoothness in the skin after application, and from the viewpoint of improving moist feeling in the skin after application.

Examples of the diester oil may include diester of dicarboxylic acid having 3 to 18 carbon atoms, and di-fatty acid ester of a polyhydric alcohol. Specific examples thereof include propylene glycol dicaprylate, neopentyl glycol dicaprate, glycol distearate, propylene glycol diisostearate, glyceryl monoisostearate monomyristate, glycerin di-2-heptylundecanoate, di-2-ethylhexyl succinate, diisopropyl sebacate, diisostearyl malate, ethylene glycol di-2-ethylhexanoate, diisobutyl adipate, di-2-heptylundecyl adipate, and di-2-ethylhexyl sebacate.

The diester oil preferably includes one or more members selected from the group consisting of propylene glycol dicaprylate and neopentyl glycol dicaprate, and more preferably neopentyl glycol dicaprate, from the viewpoint of creating fine particles of an oil containing a ceramide, from the viewpoint of improving preservation stability, from the viewpoint of improving smoothness in the skin after application, and from the viewpoint of improving moist feeling in the skin after application.

Examples of the triester oil may include tri-fatty acid ester of a trihydric or higher polyhydric alcohol and specifically include glyceryl trimyristate, glyceryl triisopalmitate, glyceryl tri-2-heptylundecanoate, trimethylolpropane triethylhexanoate, trimethylolpropane trioctanoate, glyceryl tri(caprylate-caprate), glyceryl trioleate, glyceryl tri-2-ethylhexanoate, glyceryl triisostearate, and plant oils such as olive oil, macadamia nut oil, meadowfoam oil, castor oil, safflower oil, sunflower oil, avocado oil, canola oil, apricot kernel oil, rice germ oil, and rice bran oil.

The triester oil preferably includes one or more members selected from the group consisting of glyceryl tri(caprylate-caprate), glyceryl tri-2-ethylhexanoate, and a plant oil, more preferably one or more members selected from the group consisting of glyceryl tri(caprylate-caprate) and olive oil, and further more preferably glyceryl tri(caprylate-caprate), from the viewpoint of creating fine particles of an oil containing tahe ceramide, from the viewpoint of improving preservation stability, from the viewpoint of improving smoothness in the skin after application, and from the viewpoint of improving moist feeling in the skin after application.

Examples of the tetraester oil may include tetra-fatty acid ester of a tetrahydric or higher polyhydric alcohol and specifically include pentaerythritol tetra(behenate/benzoate/ethylhexanoate), pentaerythritol tetraethylhexanoate, pentaerythritol tetraoctanoate, and pentaerythritol tetra-2-ethylhexanoate.

The ester oil preferably includes one or more members selected from the group consisting of a monoester oil, a diester oil, and a triester oil, more preferably one or more members selected from the group consisting of a monoester oil and a diester oil, and further more preferably a diester oil, from the viewpoint of creating fine particles of an oil containing a ceramide, from the viewpoint of improving preservation stability, from the viewpoint of improving smoothness in the skin after application, and from the viewpoint of improving moist feeling in the skin after application.

For an ester oil, it may contain a hydroxy group or no hydroxy group in its configuration. The ester oil preferably contains no hydroxy group in the configuration from the viewpoint of creating fine particles of an oil containing a ceramide and from the viewpoint of improving preservation stability.

Examples of the ether oil may include dialkyl ether and specifically dihexyl ether, dicaprylyl ether, and cetyl-1,3-dimethylbutyl ether. Cetyl-1,3-dimethylbutyl ether is preferably contained.

Examples of the higher alcohol may include alcohols having a linear or branched alkyl or alkenyl group having 10 to 24 carbon atoms, for example, lauryl alcohol, myristyl alcohol, isocetyl alcohol, isostearyl alcohol, 2-octyldodecanol, and oleyl alcohol.

Examples of the silicone oil may include: volatile or involatile linear dimethylpolysiloxane such as dimethylpolysiloxane (1cs), dimethylpolysiloxane (1.5cs), dimethylpolysiloxane (2cs), and dimethylpolysiloxane (6cs); branched siloxane such as methyl trimethicone, tris(trimethylsilyl)methylsilane, and tetrakis(trimethylsilyl)silane; cyclic dimethylsiloxane such as octamethylcyclotetrasiloxane, decamethylcyclopentasiloxane, and dodecamethylcyclohexasiloxane; and methylphenylpolysiloxane such as diphenylsiloxyphenyl trimethicone.

The silicone oil preferably includes one or more members selected from the group consisting of linear dimethylpolysiloxane and branched siloxane, more preferably linear dimethylpolysiloxane, further more preferably one or more members selected from the group consisting of dimethylpolysiloxane (2cs) and dimethylpolysiloxane (6cs), and even more preferably dimethylpolysiloxane (2cs), from the viewpoint of creating fine particles of an oil containing a ceramide, from the viewpoint of improving preservation stability, from the viewpoint of improving smoothness in the skin after application, and from the viewpoint of improving moist feeling in the skin after application.

Examples of the higher fatty acid may include oleic acid, isostearic acid, and undecylenic acid.

The oil agent as the component (B) preferably includes (B1) a hydrocarbon oil, (B2) a polar oil, and a silicone oil, and more preferably (B1) a hydrocarbon oil and (B2) a polar oil, from the viewpoint of creating fine particles of an oil containing a ceramide, from the viewpoint of improving preservation stability, and from the viewpoint of improving moist feeling in the skin after application.

The polar oil (B2) preferably includes one or more members selected from the group consisting of an ester oil, an ether oil, and a higher fatty acid, and more preferably an ester oil, from the viewpoint of creating fine particles of an oil containing a ceramide, from the viewpoint of improving preservation stability, from the viewpoint of improving smoothness in the skin after application, and from the viewpoint of improving moist feeling in the skin after application.

The content of the component (B1) is preferably 0.1% by mass or more, more preferably 0.3% by mass or more, further more preferably 0.5% by mass or more, even more preferably 1.5% by mass or more, and preferably 5% by mass or less, more preferably 4% by mass or less, further more preferably 3% by mass or less, even more preferably 2.5% by mass or less, in the whole composition from the viewpoint of creating fine particles of an oil containing a ceramide, from the viewpoint of improving preservation stability, from the viewpoint of improving smoothness in the skin after application, and from the viewpoint of improving moist feeling in the skin after application. The content of the component (B1) is preferably from 0.1 to 5% by mass, more preferably from 0.3 to 4% by mass, further more preferably from 0.5 to 3% by mass, even more preferably from 1.5 to 2.5% by mass, in the whole composition. The content of the component (B1) is preferably from 0.5 to 2.5% by mass in the whole composition.

The content of the component (B2) is preferably 0.05% by mass or more, more preferably 0.1% by mass or more, further more preferably 0.3% by mass or more, even more preferably 0.5% by mass or more, further more preferably 1.5% by mass or more, and preferably 3.5% by mass or less, more preferably 2.8% by mass or less, further more preferably 2.5% by mass or less, even more preferably 2.3% by mass or less, further more preferably 2.1% by mass or less, in the whole composition. The content of the component (B2) is preferably from 0.05 to 3.5% by mass, more preferably from 0.1 to 2.8% by mass, further more preferably from 0.3 to 2.5% by mass, even more preferably from 0.5 to 2.3% by mass, further more preferably from 1.5 to 2.1% by mass, in the whole composition. The content of the component (B2) is preferably from 0.3 to 3.5% by mass, more preferably from 1.5 to 2.5% by mass, in the whole composition.

In the present invention, when the oil-in-water emulsion composition contains the components (B1) and (B2) as the component (B), the mass ratio of the component (B1) to the component (B2), (B1)/(B2), is preferably 0.05 or more, more preferably 0.1 or more, further more preferably 0.5 or more, even more preferably 0.7 or more, and preferably 6 or less, more preferably 3 or less, further more preferably 2.5 or less, even more preferably 2 or less, from the viewpoint of creating fine particles of an oil containing a ceramide, from the viewpoint of improving preservation stability, from the viewpoint of improving smoothness in the skin after application, and from the viewpoint of improving moist feeling in the skin after application.

The mass ratio of the component (B1) to the component (B2), (B1)/(B2), is preferably from 0.05 to 6, more preferably from 0.1 to 3, further more preferably from 0.5 to 2.5, even more preferably from 0.7 to 2. The mass ratio of the component (B1) to the component (B2), (B1)/(B2), is preferably from 0.5 to 6, more preferably from 0.7 to 3, further more preferably from 0.7 to 2.5.

One or more members can be used as the component (B). The content thereof is preferably 0.15% by mass or more, more preferably 1% by mass or more, further more preferably 3% by mass or more, even more preferably 5% by mass or more, and preferably 9% by mass or less, more preferably 7.5% by mass or less, further more preferably 7% by mass or less, even more preferably 6.5% by mass or less, in the whole composition from the viewpoint of creating fine particles of an oil containing a ceramide, from the viewpoint of improving preservation stability, from the viewpoint of improving smoothness in the skin after application, and from the viewpoint of improving moist feeling in the skin after application.

The content of the component (B) is preferably from 0.15 to 9% by mass, more preferably from 1 to 7.5% by mass, further more preferably from 3 to 7% by mass, even more preferably from 5 to 6.5% by mass, in the whole composition. The content of the component (B) is preferably from 1 to 9% by mass in the whole composition.

In the present invention, the mass ratio of the component (A) to the component (B), (A)/(B), is preferably 0.05 or more, more preferably 0.1 or more, further more preferably 0.12 or more, even more preferably 0.15 or more, and preferably 1 or less, more preferably 0.4 or less, further more preferably 0.3 or less, even more preferably 0.2 or less, from the viewpoint of creating fine particles of an oil containing a ceramide, from the viewpoint of improving preservation stability, from the viewpoint of improving smoothness in the skin after application, and from the viewpoint of improving moist feeling in the skin after application.

The mass ratio of the component (A) to the component (B), (A)/(B), is preferably from 0.05 to 1, more preferably from 0.1 to 0.4, further more preferably from 0.12 to 0.3, even more preferably from 0.15 to 0.2. The mass ratio of the component (A) to the component (B), (A)/(B), is preferably from 0.05 to 0.4, more preferably from 0.05 to 0.3.

The component (C) is a nonionic surfactant having HLB of less than 10.

In this context, the HLB (hydrophilic-lipophilic balance) refers to the molecular weight of a hydrophilic group moiety which accounts for the total molecular weight of the surfactant. In the present invention, the HLB of the nonionic surfactant is determined according to the equation of Griffin.

The nonionic surfactant having HLB of less than 10 is not limited as long as the nonionic surfactant can be used in usual cosmetics. Examples thereof include polyoxyethylene fatty acid ester, polyoxyethylene sorbitan fatty acid ester, polyoxyethylene alkyl ether, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene hydrogenated castor oil fatty acid ester, polyoxyethylene phytostanol ether, polyoxyethylene phytosterol ether, polyoxyethylene cholestanol ether, polyoxyethylene cholesteryl ether, silicone-based surfactants such as polyether-modified silicone, sucrose fatty acid ester, glycerin fatty acid ester, alkyl saccharide, alkyl glyceryl ether, and sorbitan fatty acid ester.

The nonionic surfactant having HLB of less than 10 has HLB of preferably 7 or less, more preferably 5 or less, from the viewpoint of creating fine particles of an oil containing a ceramide and from the viewpoint of improving preservation stability. The HLB is preferably 1 or more, more preferably 3 or more.

Among them, the component (C) preferably includes one or more members selected from the group consisting of polyether-modified silicone, glycerin fatty acid ester, alkyl glyceryl ether, and sorbitan fatty acid ester, more preferably one or more members selected from the group consisting of glycerin fatty acid ester and sorbitan fatty acid ester, further more preferably sorbitan fatty acid ester, from the viewpoint of creating fine particles of an oil containing a ceramide and from the viewpoint of improving preservation stability.

One or more members can be used as the component (C). The content thereof is preferably 0.1% by mass or more, more preferably 0.4% by mass or more, further more preferably 0.5% by mass or more, even more preferably 0.55% by mass or more, and preferably 3% by mass or less, more preferably 2.5% by mass or less, further more preferably 1.8% by mass or less, even more preferably 1.4% by mass or less, in the whole composition from the viewpoint of creating fine particles of an oil containing a ceramide and from the viewpoint of improving preservation stability.

The content of the component (C) is preferably from 0.1 to 3% by mass, more preferably from 0.4 to 2.5% by mass, further more preferably from 0.5 to 1.8% by mass, even more preferably from 0.55 to 1.4% by mass, in the whole composition. The content of the component (C) is preferably from 0.1 to 2.5, in the whole composition.

In the present invention, the mass ratio of the component (A) to the component (C), (A)/(C), is preferably 0.05 or more, more preferably 0.1 or more, further more preferably 0.5 or more, even more preferably 0.6 or more, further more preferably 0.8 or more, and preferably 10 or less, more preferably 5 or less, further more preferably 2.5 or less, even more preferably 2.2 or less, further more preferably 1.8 or less, from the viewpoint of creating fine particles of an oil containing a ceramide, from the viewpoint of improving preservation stability and improving smoothness in the skin after application, and from the viewpoint of improving moist feeling in the skin after application.

The mass ratio of the component (A) to the component (C), (A)/(C), is preferably from 0.05 to 10, more preferably from 0.1 to 5, further more preferably from 0.5 to 2.5, even more preferably from 0.6 to 2.2, further more preferably from 0.8 to 1.8. The mass ratio of the component (A) to the component (C), (A)/(C), is preferably from 0.1 to 10.

In the present invention, the mass ratio of the component (B) to the component (C), (B)/(C), is preferably 0.05 or more, more preferably 1 or more, further more preferably 3 or more, even more preferably 3.5 or more, further more preferably 4.5 or more, and preferably 40 or less, more preferably 20 or less, further more preferably 15 or less, even more preferably 12 or less, further more preferably 10 or less, from the viewpoint of creating fine particles of an oil containing a ceramide, from the viewpoint of improving preservation stability and improving smoothness in the skin after application, and from the viewpoint of improving moist feeling in the skin after application.

The mass ratio of the component (B) to the component (C), (B)/(C), is preferably from 0.05 to 40, more preferably from 1 to 20, further more preferably from 3 to 15, even more preferably from 3.5 to 12, and further more preferably from 4.5 to 10. The mass ratio of the component (B) to the component (C), (B)/(C), is preferably from 1 to 40.

In the present invention, the content of the water as the component (D) is preferably 50% by mass or more, more preferably 60% by mass or more, further more preferably 70% by mass or more, and preferably 95% by mass or less, more preferably 90% by mass or less, further more preferably 88% by mass or less, in the whole composition from the viewpoint of creating fine particles of an oil containing a ceramide, from the viewpoint of improving preservation stability, from the viewpoint of improving smoothness in the skin after application, and from the viewpoint of improving moist feeling in the skin after application. The content of the water as the component (D) is preferably from 50 to 95% by mass, more preferably from 60 to 90% by mass, further more preferably from 70 to 88% by mass, in the whole composition.

The oil-in-water emulsion composition of the present invention can further contain (E) a nonionic surfactant having HLB of 10 or more, which can create fine particles of an oil containing a ceramide and more improve preservation stability.

The nonionic surfactant as the component (E) preferably has HLB of from 11 to 16, more preferably HLB of from 12 to 15, from the viewpoint of creating fine particles of an oil containing a ceramide and from the viewpoint of improving preservation stability.

Examples of the nonionic surfactant as the component (E) may include polyoxyethylene hydrogenated castor oil, polyoxyethylene sorbitan fatty acid ester, polyoxyethylene alkyl ether, polyoxyethylene fatty acid ester, polyoxyethylene alkanol ether, polyglycerin fatty acid ester, and sucrose fatty acid ester. Among them, the component (E) preferably includes one or more members selected from the group consisting of polyoxyethylene hydrogenated castor oil and polyoxyethylene sorbitan fatty acid ester, more preferably polyoxyethylene sorbitan fatty acid ester, from the viewpoint of creating fine particles of an oil containing a ceramide and from the viewpoint of improving preservation stability.

In the polyoxyethylene hydrogenated castor oil, the average number of moles of an added polyoxyethylene chain constituting the polyoxyethylene hydrogenated castor oil is preferably from 25 to 100, more preferably from 35 to 85, further more preferably from 45 to 75, even more preferably from 55 to 65, especially preferably 60, from the viewpoint of creating fine particles of an oil containing a ceramide and from the viewpoint of improving preservation stability.

In the polyoxyethylene sorbitan fatty acid ester, the average number of moles of an added polyoxyethylene chain constituting the polyoxyethylene sorbitan fatty acid ester is preferably from 10 to 30, more preferably from 15 to 25, further more preferably 20, from the viewpoint of creating fine particles of an oil containing a ceramide and from the viewpoint of improving preservation stability. In the polyoxyethylene sorbitan fatty acid ester, the number of carbon atoms in the alkyl group constituting the polyoxyethylene sorbitan fatty acid ester is preferably from 10 to 25, more preferably from 16 to 20, further more preferably 18, from the viewpoint of creating fine particles of an oil containing a ceramide and from the viewpoint of improving preservation stability.

One or more nonionic surfactants can be used (in combination) as the component (E). The content thereof is preferably 0.5% by mass or more, more preferably 1% by mass or more, further more preferably 1.4% by mass or more, and preferably 3% by mass or less, more preferably 2.5% by mass or less, further more preferably 2% by mass or less, in the whole composition from the viewpoint of creating fine particles of an oil containing a ceramide and from the viewpoint of improving preservation stability. The content of the component (E) is preferably from 0.5 to 3% by mass, more preferably from 1 to 2.5% by mass, further more preferably from 1.4 to 2% by mass, in the whole composition.

The oil-in-water emulsion composition of the present invention can further contain an ionic surfactant, which can create fine particles of an oil containing a ceramide and improve preservation stability.

Examples of the ionic surfactant may include anionic surfactants, cationic surfactants, and ampholytic surfactants.

Examples of the anionic surfactant may include: fatty acids having 12 to 22 carbon atoms and salts thereof, such as sodium laurate, potassium palmitate, and arginine stearate;
C12 to C22 alkyl sulfuric acid ester and salts thereof, such as sodium lauryl sulfate, potassium lauryl sulfate, and sodium cetyl sulfate;
C12 to C22 alkyl ether sulfuric acid ester and salts thereof, such as triethanolamine polyoxyethylene lauryl sulfate;
N-C12 to C22 acyl sarcosine and salts thereof, such as sodium lauroyl sarcosine;
C12 to C22 alkyl phosphoric acid and salts thereof, such as sodium monostearyl phosphate;
polyoxyethylene C12 to C22 alkyl ether phosphoric acid and salts thereof, such as sodium polyoxyethylene oleyl ether phosphate and sodium polyoxyethylene stearyl ether phosphate;
C12 to C24 dialkyl sulfosuccinic acid and salts thereof, such as sodium di-2-ethylhexyl sulfosuccinate; N-alkyloyl methyltaurine having 12 to 22 carbon atoms and salts thereof, such as sodium N-stearoyl-N-methyltaurine; and
N-C12 to C22 acyl glutamic acid and salts thereof, such as sodium dilauroyl glutamate, monosodium N-lauroyl glutamate, sodium N-stearoyl-L-glutamate, arginine N-stearoyl-L-glutamate, sodium N-stearoyl glutamate, and sodium N-myristoyl-L-glutamate.

The N-alkyloyl methyltaurine having 12 to 22 carbon atoms or the salt thereof is preferably sodium N-stearoyl-N-methyltaurine from the viewpoint of creating fine particles of an oil containing a ceramide and from the viewpoint of improving preservation stability.

The N-C12 to C22 acyl glutamic acid or the salt thereof is preferably sodium N-stearoyl-L-glutamate from the viewpoint of creating fine particles of an oil containing a ceramide and from the viewpoint of improving preservation stability.

Sodium N-stearoyl-N-methyltaurine is also referred to as stearoyl methyltaurine, and sodium N-stearoyl-L-glutamate is also referred to as Na stearoyl glutamate.

The cationic surfactant is preferably quaternary ammonium salt. Examples thereof include alkyl trimethylammonium chloride such as stearyl trimethylammonium chloride and behenyl trimethylammonium chloride, dialkyl dimethylammonium chloride, trialkyl methylammonium chloride, and alkylamine salts.

Examples of the ampholytic surfactant may include, alkyl dimethylamine oxide, alkyl carboxybetaine, alkyl sulfobetaine, amide amino acid salts, and alkylamide propylbetaine. Among them, alkylamide propylbetaine is preferred.

The ionic surfactant preferably contains an anionic surfactant from the viewpoint of creating fine particles of an oil containing a ceramide and from the viewpoint of improving preservation stability.

The anionic surfactant preferably includes one or more members selected from the group consisting of N-alkyloyl methyltaurine having 12 to 22 carbon atoms or a salt thereof, and N-C12 to C22 acyl glutamic acid or a salt thereof, more preferably N-alkyloyl methyltaurine having 12 to 22 carbon atoms or a salt thereof, and further more preferably N-stearoyl-N-methyltaurine or a salt thereof, from the viewpoint of creating fine particles of an oil containing a ceramide and from the viewpoint of improving preservation stability.

One or more ionic surfactants can be used (in combination). The content thereof is preferably 0.05% by mass or more, more preferably 0.1% by mass or more, further more preferably 0.13% by mass or more, even more preferably 0.15% by mass or more, further more preferably 0.18% by mass or more, and preferably 2% by mass or less, more preferably 1% by mass or less, further more preferably 0.8% by mass or less, even more preferably 0.5% by mass or less, further more preferably 0.45% by mass or less, in the whole composition from the viewpoint of creating fine particles of an oil containing a ceramide and from the viewpoint of improving preservation stability. The content of the ionic surfactant is preferably from 0.05 to 2% by mass, more preferably from 0.1 to 1% by mass, further more preferably from 0.13 to 0.8% by mass, even more preferably from 0.15 to 0.5% by mass, further more preferably from 0.18 to 0.45% by mass, in the whole composition. The content of the ionic surfactant is preferably from 0.1 to 2% by mass, more preferably from 0.1 to 0.8% by mass.

The oil-in-water emulsion composition of the present invention can further contain a polyhydric alcohol, which can create fine particles of an oil containing a ceramide and improve preservation stability. The polyhydric alcohol is a compound having two or more hydroxy groups in the molecule. Any polyhydric alcohol may be used as long as the polyhydric alcohol can be used in usual cosmetics.

Examples of the dihydric alcohol may include ethylene glycol, diethylene glycol, triethylene glycol, polyethylene glycol, propylene glycol, dipropylene glycol, tripropylene glycol, polypropylene glycol, 1,3-butylene glycol, 1,3-propanediol, and pentanediol. Examples of the trihydric alcohol may include glycerin and trimethylolpropane. Examples of the tetrahydric alcohol may include diglycerin and erythritol. Examples of the pentahydric or higher polyhydric alcohol may include: polyglycerin such as triglycerin; and saccharides and sugar alcohols, such as glucose, maltose, maltitol, sucrose, xylitol, sorbitol, maltitol, polyoxyethylene methyl glucoside, polyoxyethylene ethyl glucoside, and polyoxyethylene propylene glucoside.

Among them, the polyhydric alcohol preferably includes one or more members selected from the group consisting of a dihydric alcohol and a trihydric alcohol, more preferably a dihydric alcohol, and further more preferably one or more members selected from the group consisting of 1,3-butylene glycol and dipropylene glycol, from the viewpoint of creating fine particles of an oil containing a ceramide and from the viewpoint of improving preservation stability.

One or more polyhydric alcohols can be used (in combination). The content thereof is preferably 1% by mass or more, more preferably 1.3% by mass or more, further more preferably 1.5% by mass or more, even more preferably 1.8% by mass or more, and preferably 30% by mass or less, more preferably 20% by mass or less, further more preferably 15% by mass or less, even more preferably 12% by mass or less, in the whole composition from the viewpoint of creating fine particles of an oil containing a ceramide and from the viewpoint of improving preservation stability. The content of the polyhydric alcohol is preferably from 1 to 30% by mass, more preferably from 1.3 to 20% by mass, further more preferably from 1.5 to 15% by mass, even more preferably from 1.8 to 12% by mass, in the whole composition.

When the oil-in-water emulsion composition contains a dihydric alcohol as the polyhydric alcohol, one or more dihydric alcohols can be used (in combination). The content thereof is preferably 1% by mass or more, more preferably 1.3% by mass or more, further more preferably 1.5% by mass or more, even more preferably 1.8% by mass or more, and preferably 30% by mass or less, more preferably 20% by mass or less, further more preferably 15% by mass or less, even more preferably 10% by mass or less, in the whole composition from the viewpoint of creating fine particles of an oil containing a ceramide and from the viewpoint of improving preservation stability. The content of the dihydric alcohol is preferably from 1 to 30% by mass, more preferably from 1.3 to 20% by mass, further more preferably from 1.5 to 15% by mass, even more preferably from 1.8 to 10% by mass, in the whole composition.

The oil-in-water emulsion composition of the present invention can contain components conventionally used in cosmetics, for example, an oil component other than the component (B), a water-soluble polymer, an antioxidant, a fragrance, an antiseptic, a pH adjuster, a blood circulation promoter, a cooling agent, an antiperspirant, a germicide, a skin activator, a moisturizing agent other than the polyhydric alcohol, an algefacient, and a colorant, in addition to the components described above.

A method for producing the oil-in-water emulsion composition of the present invention includes: a step 1 of obtaining an oil phase containing the component (A), the component (B), and the component (C); and a step 2 of mixing the oil phase obtained in the step 1 with an aqueous phase containing the component (D).

The oil-in-water emulsion composition of the present invention preferably contains a dihydric alcohol from the viewpoint of obtaining a fine emulsion. In this case, the production method is preferably a method for producing the oil-in-water emulsion composition, including: a step 1 of obtaining an oil phase containing the component (A), the component (B), the component (C), the dihydric alcohol, and a portion of the component (D); and a step 2 of mixing the oil phase obtained in the step 1 with an aqueous phase containing the remaining portion of the component (D).

In the step 1, preferably, the oil phase contains the dihydric alcohol and a portion of the water as the component (D), and the oil phase obtained in the step 1 is visually transparent (at a mixing temperature), from the viewpoint of obtaining a fine emulsion. In this respect, the content of the dihydric alcohol in the oil phase of the step 1 is preferably 5% by mass or more, more preferably 8% by mass or more, from the viewpoint of obtaining a fine emulsion, and preferably 30% by mass or less, more preferably 25% by mass or less, from the same viewpoint as above. The content of the water as the component (D) in the oil phase of the step 1 is preferably 2% by mass or more, more preferably 5% by mass or more, from the viewpoint of obtaining a fine emulsion, and preferably 10% by mass or less, more preferably 8% by mass or less, from the same viewpoint as above.

The content of the component (A) in the oil phase of the step 1 is preferably 1% by mass or more, more preferably 2% by mass or more, from the viewpoint of improving moist feeling in the skin after application, and preferably 15% by mass or less, more preferably 13% by mass or less, from the viewpoint of obtaining a fine emulsion. The content of the component (B) in the oil phase of the step 1 is preferably 20% by mass or more, more preferably 30% by mass or more, from the viewpoint of improving moist feeling in the skin after application, and preferably 90% by mass or less, more preferably 80% by mass or less, from the viewpoint of obtaining a fine emulsion. The content of the component (C) in the oil phase of the step 1 is preferably 2% by mass or more, more preferably 3% by mass or more, from the viewpoint of obtaining a fine emulsion, and preferably 30% by mass or less, more preferably 20% by mass or less, from the viewpoint of obtaining a fine emulsion.

The oil phase of the step 1 can contain the component (E) and other surfactants as additional components. The aqueous phase can contain a polyhydric alcohol, other than the dihydric alcohol, such as glycerin as an additional component.

In the step 2, the oil phase obtained in the step 1 and the aqueous phase are preferably heated to preferably 60°C or higher, more preferably 70°C or higher, and preferably 100°C or lower, and mixed, from the viewpoint of obtaining a fine emulsion. The oil phase of the step 1 is preferably added to the aqueous phase and mixed, from the viewpoint of obtaining a fine emulsion.

In the step 2, the mix ratio by mass between the oil phase and the aqueous phase (oil phase/aqueous phase) is preferably 2/98 or more, more preferably 5/95 or more, from the viewpoint of productivity, and preferably 40/60 or less, more preferably 30/70 or less, from the viewpoint of obtaining a fine emulsion.

After the oil phase and the aqueous phase are mixed together, it is preferable to stir the mixture for 10 minutes or longer and 2 hours or shorter, and then cooled to room temperature. The mixing can be performed by using a conventional stirring apparatus with a stirring blade.

In the oil-in-water emulsion composition of the present invention, the Z-average particle size of emulsion particles is from 0.05 to 0.3 µm, preferably from 0.05 to 0.25 µm, more preferably from 0.05 to 0.2 µm, further more preferably from 0.05 to 0.15 µm, from the viewpoint of creating fine particles of an oil containing a ceramide and improving preservation stability.

In the present invention, the Z-average particle size of the emulsion particles can be measured at 25°C by using each oil-in-water emulsion composition diluted 20-fold and Zetasizer Nano Z manufactured by Malvern Panalytical Ltd. A cell of 10 × 10 × 45 mm manufactured by Sarstedt K.K. is used.

A fine particle size can render the oil-in-water emulsion composition of the present invention having low viscosity. As a result, when the oil-in-water emulsion composition of the present invention is applied as a makeup remover mentioned later, cotton is easily impregnated with the oil-in-water emulsion composition by putting the oil-in-water emulsion composition onto the cotton, or makeup stains can be removed by wiping the face with the cotton, without the cotton slipping on the skin.

The oil-in-water emulsion composition of the present invention is suitable for use as an oil-in-water emulsion cosmetic. The oil-in-water emulsion composition of the present invention can be applied as, for example, a skincare cosmetic such as a lotion, an emulsion, or a serum, or a skin cosmetic such as a makeup remover. The oil-in-water emulsion composition of the present invention is suitable as a makeup remover because the oil-in-water emulsion composition has fine particles of an oil containing a ceramide and is put onto cotton so as to easily impregnate the cotton.

The oil-in-water emulsion composition of the present invention can be used by taking the composition in the hand and applying it to the skin with fingers. The oil-in-water emulsion composition of the present invention can also be used by impregnating cotton or a sheet with the oil-in-water emulsion composition and wiping the skin. The oil-in-water emulsion composition, when used as a makeup remover, is suitably used by impregnating cotton with the composition.

For the oil-in-water emulsion composition of the present invention to be used as a makeup remover, the viscosity at 25°C is preferably from 10 to 3 000 mPa·s, more preferably from 10 to 1 000 mPa·s, further more preferably from 10 to 400 mPa·s, from the viewpoint of easily impregnating cotton with the oil-in-water emulsion composition.

The viscosity of the oil-in-water emulsion composition of the present invention can be measured by using a B type viscometer under measurement conditions given below. In all the cases, a measurement temperature is 25°C.
Viscosity of 1 mPa·s or more and less than 200 mPa·s: rotor No. 1, 30 rpm, 30 sec,
Viscosity of 200 mPa·s or more and less than 500 mPa·s: rotor No. 1, 12 rpm, 30 sec,
Viscosity of 500 mPa·s or more and less than 2 500 mPa·s: rotor No. 2, 12 rpm, 30 sec, and
Viscosity of 2 500 mPa·s or more and less than 10 000 mPa·s: rotor No. 3, 12 rpm, 30 sec.

In relation to the embodiments mentioned above, the present invention further discloses the following compositions, production methods, and the like.
<1> An oil-in-water emulsion composition comprising the following components (A), (B), (C), and (D):
   (A) a ceramide,
   (B) an oil agent in a liquid state at 25°C,
   (C) a nonionic surfactant having HLB of less than 10, and
   (D) water, wherein
   a Z-average particle size of emulsion particles is from 0.05 to 0.3 µm.
<2> The oil-in-water emulsion composition according to <1>, wherein the ceramide as the component (A) preferably comprises one or more members selected from the group consisting of natural ceramide, sphingosines, and compounds of the formulas (1) and (2): 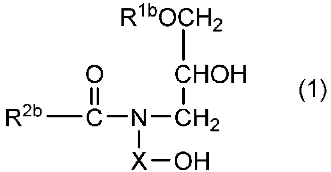 wherein R^{1b} represents a hydrocarbon group having 10 to 26 carbon atoms, R^{2b} represents a hydrocarbon group having 9 to 25 carbon atoms, and X represents -(CH₂)ₙ-(wherein n represents an integer of from 2 to 6), and 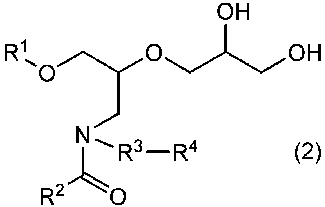 wherein R¹ and R² are the same or different and each represent an optionally hydroxylated hydrocarbon group having 1 to 40 carbon atoms, R³ represents an alkylene group having 1 to 6 carbon atoms or a single bond, and R⁴ represents a hydrogen atom, an alkoxy group having 1 to 12 carbon atoms, or 2,3-dihydroxypropyloxy group, on the proviso that when R³ is a single bond, R⁴ is a hydrogen atom, more preferably comprises one or more members selected from the group consisting of natural ceramide, sphingosines, and a compound of the formula (1), further more preferably comprises one or more members selected from the group consisting of natural ceramide, phytosphingosine, and N-(hexadecyloxyhydroxypropyl)-N-hydroxyethylhexadecanamide, and even more preferably comprises N-(hexadecyloxyhydroxypropyl)-N-hydroxyethylhexadecanamide.
<3> The oil-in-water emulsion composition according to <1> or <2>, wherein a content of the component (A) is preferably 0.5% by mass or more, more preferably 0.6% by mass or more, further more preferably 0.7% by mass or more, even more preferably 0.8% by mass or more, and preferably 3% by mass or less, more preferably 2% by mass or less, further more preferably 1.8% by mass or less, even more preferably 1.3% by mass or less, in the whole composition.
<4> The oil-in-water emulsion composition according to any one of <1> to <3>, wherein the oil agent as the component (B) is preferably one or more members selected from the group consisting of a hydrocarbon oil, an ester oil, an ether oil, a higher alcohol, a silicone oil, and a higher fatty acid,
   more preferably comprises (B1) a hydrocarbon oil, (B2) a polar oil, and a silicone oil, and further more preferably comprises (B1) a hydrocarbon oil and (B2) a polar oil.
<5> The oil-in-water emulsion composition according to <4>, wherein a content of the component (B1) is preferably 0.1% by mass or more, more preferably 0.3% by mass or more, further more preferably 0.5% by mass or more, even more preferably 1.5% by mass or more, and preferably 5% by mass or less, more preferably 4% by mass or less, further more preferably 3% by mass or less, even more preferably 2.5% by mass or less, in the whole composition.
<6> The oil-in-water emulsion composition according to <4> or <5>, wherein a content of the component (B2) is preferably 0.05% by mass or more, more preferably 0.1% by mass or more, further more preferably 0.3% by mass or more, even more preferably 0.5% by mass or more, further more preferably 1.5% by mass or more, and preferably 3.5% by mass or less, more preferably 2.8% by mass or less, further more preferably 2.5% by mass or less, even more preferably 2.3% by mass or less, further more preferably 2.1% by mass or less, in the whole composition.
<7> The oil-in-water emulsion composition according to any one of <4> to <6>, wherein the oil-in-water emulsion composition preferably comprises the components (B1) and (B2) as the component (B), and a mass ratio of the component (B1) to the component (B2), (B1)/(B2), is preferably 0.05 or more, more preferably 0.1 or more, further more preferably 0.5 or more, even more preferably 0.7 or more, and preferably 6 or less, more preferably 3 or less, further more preferably 2.5 or less, even more preferably 2 or less.
<8> The oil-in-water emulsion composition according to any one of <1> to <7>, wherein a content of the component (B) is preferably 0.15% by mass or more, more preferably 1% by mass or more, further more preferably 3% by mass or more, even more preferably 5% by mass or more, and preferably 9% by mass or less, more preferably 7.5% by mass or less, further more preferably 7% by mass or less, even more preferably 6.5% by mass or less, in the whole composition.
<9> The oil-in-water emulsion composition according to any one of <1> to <8>, wherein a mass ratio of the component (A) to the component (B), (A)/(B), is preferably 0.05 or more, more preferably 0.1 or more, further more preferably 0.12 or more, even more preferably 0.15 or more, and preferably 1 or less, more preferably 0.4 or less, further more preferably 0.3 or less, even more preferably 0.2 or less.
<10> The oil-in-water emulsion composition according to any one of <1> to <9>, wherein the component (C) has HLB of preferably 7 or less, more preferably 5 or less, and HLB of preferably 1 or more, more preferably 3 or more.
<11> The oil-in-water emulsion composition according to any one of <1> to <10>, wherein the component (C) preferably comprises one or more members selected from the group consisting of polyether-modified silicone, glycerin fatty acid ester, alkyl glyceryl ether, and sorbitan fatty acid ester, more preferably one or more members selected from the group consisting of glycerin fatty acid ester and sorbitan fatty acid ester, and further more preferably sorbitan fatty acid ester.
<12> The oil-in-water emulsion composition according to any one of <1> to <11>, wherein a content of the component (C) is preferably 0.1% by mass or more, more preferably 0.4% by mass or more, further more preferably 0.5% by mass or more, even more preferably 0.55% by mass or more, and preferably 3% by mass or less, more preferably 2.5% by mass or less, further more preferably 1.8% by mass or less, even more preferably 1.4% by mass or less, in the whole composition.
<13> The oil-in-water emulsion composition according to any one of <1> to <12>, wherein a mass ratio of the component (A) to the component (C), (A)/(C), is preferably 0.05 or more, more preferably 0.1 or more, further more preferably 0.5 or more, even more preferably 0.6 or more, further more preferably 0.8 or more, and preferably 10 or less, more preferably 5 or less, further more preferably 2.5 or less, even more preferably 2.2 or less, further more preferably 1.8 or less.
<14> The oil-in-water emulsion composition according to any one of <1> to <13>, wherein a mass ratio of the component (B) to the component (C), (B)/(C), is preferably 0.05 or more, more preferably 1 or more, further more preferably 3 or more, even more preferably 3.5 or more, further more preferably 4.5 or more, and preferably 40 or less, more preferably 20 or less, further more preferably 15 or less, even more preferably 12 or less, further more preferably 10 or less.
<15> The oil-in-water emulsion composition according to any one of <1> to <14>, wherein a content of the water as the component (D) is preferably 50% by mass or more, more preferably 60% by mass or more, further more preferably 70% by mass or more, and preferably 95% by mass or less, more preferably 90% by mass or less, further more preferably 88% by mass or less, in the whole composition.
<16> The oil-in-water emulsion composition according to any one of <1> to <15>, preferably further comprising (E) a nonionic surfactant having HLB of 10 or more, more preferably HLB of from 11 to 16, further more preferably HLB of from 12 to 15.
<17> The oil-in-water emulsion composition according to <16>, wherein the nonionic surfactant as the component (E) preferably comprises one or more members selected from the group consisting of polyoxyethylene hydrogenated castor oil and polyoxyethylene sorbitan fatty acid ester, and more preferably polyoxyethylene sorbitan fatty acid ester.
<18> The oil-in-water emulsion composition according to <17>, wherein in the polyoxyethylene hydrogenated castor oil, an average number of moles of an added polyoxyethylene chain constituting the polyoxyethylene hydrogenated castor oil is preferably from 25 to 100, more preferably from 35 to 85, further more preferably from 45 to 75, even more preferably from 55 to 65, especially preferably 60.
<19> The oil-in-water emulsion composition according to <17>, wherein in the polyoxyethylene sorbitan fatty acid ester, an average number of moles of an added polyoxyethylene chain constituting the polyoxyethylene sorbitan fatty acid ester is preferably from 10 to 30, more preferably from 15 to 25, further more preferably 20, and the number of carbon atoms in the alkyl group is preferably from 10 to 25, more preferably from 16 to 20, further more preferably 18.
<20> The oil-in-water emulsion composition according to any one of <16> to <19>, wherein a content of the component (E) is preferably 0.5% by mass or more, more preferably 1% by mass or more, further more preferably 1.4% by mass or more, and preferably 3% by mass or less, more preferably 2.5% by mass or less, further more preferably 2% by mass or less, in the whole composition.
<21> The oil-in-water emulsion composition according to any one of <1> to <20>, preferably further comprising an ionic surfactant, more preferably comprising an anionic surfactant,
   further more preferably comprising one or more members selected from the group consisting of N-alkyloyl methyltaurine having 12 to 22 carbon atoms or a salt thereof, and N-C12 to C22 acyl glutamic acid or a salt thereof,
   even more preferably comprising N-alkyloyl methyltaurine having 12 to 22 carbon atoms or a salt thereof, and further more preferably comprising N-stearoyl-N-methyltaurine or a salt thereof.
   <22> The oil-in-water emulsion composition according to <21>, wherein a content of the ionic surfactant is preferably 0.05% by mass or more, more preferably 0.1% by mass or more, further more preferably 0.13% by mass or more, even more preferably 0.15% by mass or more, further more preferably 0.18% by mass or more, and preferably 2% by mass or less, more preferably 1% by mass or less, further more preferably 0.8% by mass or less, even more preferably 0.5% by mass or less, further more preferably 0.45% by mass or less, in the whole composition.
<23> The oil-in-water emulsion composition according to any one of <1> to <22>, preferably further comprising a polyhydric alcohol, more preferably comprising one or more members selected from the group consisting of a dihydric alcohol and a trihydric alcohol, further more preferably comprising a dihydric alcohol, and even more preferably comprising one or more members selected from the group consisting of 1,3-butylene glycol and dipropylene glycol.
<24> The oil-in-water emulsion composition according to <23>, wherein a content of the polyhydric alcohol is preferably 1% by mass or more, more preferably 1.3% by mass or more, further more preferably 1.5% by mass or more, even more preferably 1.8% by mass or more, and preferably 30% by mass or less, more preferably 20% by mass or less, further more preferably 15% by mass or less, even more preferably 12% by mass or less, in the whole composition.
<25> The oil-in-water emulsion composition according to <23> or <24>, wherein when the polyhydric alcohol comprises a dihydric alcohol, a content of the dihydric alcohol is preferably 1% by mass or more, more preferably 1.3% by mass or more, further more preferably 1.5% by mass or more, even more preferably 1.8% by mass or more, and preferably 30% by mass or less, more preferably 20% by mass or less, further more preferably 15% by mass or less, even more preferably 10% by mass or less, in the whole composition.
<26> The oil-in-water emulsion composition according to any one of <1> to <25>, wherein the Z-average particle size of the emulsion particles is preferably from 0.05 to 0.25 µm, more preferably from 0.05 to 0.2 µm, further more preferably from 0.05 to 0.15 µm.
<27> The oil-in-water emulsion composition according to any one of <1> to <26> which is preferably an oil-in-water emulsion cosmetic.
<28> The oil-in-water emulsion composition according to any one of <1> to <27> which is used by taking the oil-in-water emulsion composition in the hand and applying it to the skin with fingers.
<29> The oil-in-water emulsion composition according to any one of <1> to <27> which is used by impregnating cotton or a sheet with the oil-in-water emulsion composition and wiping the skin.
<30> A method for producing the oil-in-water emulsion composition according to any one of <1> to <29>, comprising: a step 1 of obtaining an oil phase containing the component (A), the component (B), and the component (C); and a step 2 of mixing the oil phase obtained in the step 1 with an aqueous phase containing the component (D).
<31> The method for producing the oil-in-water emulsion composition according to <30>, wherein the oil-in-water emulsion composition comprises a dihydric alcohol, and the method comprises: a step 1 of obtaining an oil phase containing the component (A), the component (B), the component (C), the dihydric alcohol, and a portion of the component (D); and a step 2 of mixing the oil phase obtained in the step 1 with an aqueous phase containing the remaining portion of the component (D).
<32> The method for producing the oil-in-water emulsion composition according to <31>, wherein a content of the dihydric alcohol is preferably 5% by mass or more, more preferably 8% by mass or more, and preferably 30% by mass or less, more preferably 25% by mass or less, in the oil phase of the step 1.
<33> The method for producing the oil-in-water emulsion composition according to any one of <30> to <32>, wherein a content of the water as the component (D) is preferably, 2% by mass or more, more preferably 5% by mass or more, and preferably 10% by mass or less, more preferably 8% by mass or less, in the oil phase of the step 1.
<34> The method for producing the oil-in-water emulsion composition according to any one of <30> to <33>, wherein a content of the component (A) is preferably 1% by mass or more, more preferably 2% by mass or more, and preferably 15% by mass or less, more preferably 13% by mass or less, in the oil phase of the step 1.
<35> The method for producing the oil-in-water emulsion composition according to any one of <30> to <34>, wherein a content of the component (B) is preferably 20% by mass or more, more preferably 30% by mass or more, and preferably 90% by mass or less, more preferably 80% by mass or less, in the oil phase of the step 1.
<36> The method for producing the oil-in-water emulsion composition according to any one of <30> to <35>, wherein a content of the component (C) is preferably 2% by mass or more, more preferably 3% by mass or more, and preferably 30% by mass or less, more preferably 20% by mass or less, in the oil phase of the step 1.
<37> The method for producing the oil-in-water emulsion composition according to any one of <30> to <36>, wherein in the step 2, the oil phase obtained in the step 1 and the aqueous phase are heated to preferably 60°C or higher, more preferably 70°C or higher, and preferably 100°C or lower, and mixed.
<38> The method for producing the oil-in-water emulsion composition according to any one of <30> to <37>, wherein in the step 2, a mix ratio by mass between the oil phase and the aqueous phase (oil phase/aqueous phase) is preferably 2/98 or more, more preferably 5/95 or more, and preferably 40/60 or less, more preferably 30/70 or less.
<39> An oil-in-water emulsion composition comprising the following components (A), (B), (C), and (D):
   (A) a ceramide,
   (B) an oil agent in a liquid state at 25°C,
   (C) a nonionic surfactant having HLB of less than 10, and
   (D) water, and
   an anionic surfactant, wherein
   a Z-average particle size of emulsion particles is from 0.05 to 0.3 µm.
<40> An oil-in-water emulsion composition comprising the following components (A), (B), (C), and (D):
   (A) a ceramide,
   (B) an oil agent in a liquid state at 25°C,
   (C) a nonionic surfactant having HLB of less than 10, and
   (D) water, wherein
      a particle size of emulsion particles is from 0.05 to 0.3 µm,
      the component (B) comprises
         (B1) a hydrocarbon oil, and
         (B2) 0.05 to 3.5% by mass of a polar oil, and
      a mass ratio of the component (A) to the component (C), (A)/(C), is from 0.05 to 10.
<41> An oil-in-water emulsion composition comprising the following components (A), (B), (C), and (D):
   (A) a ceramide,
   (B) an oil agent in a liquid state at 25°C,
   (C) a nonionic surfactant having HLB of less than 10, and
   (D) water, wherein
      an average particle size of emulsion particles is from 0.05 to 0.3 µm, and
      the oil-in-water emulsion composition further comprises 0.05 to 2% by mass of one or more members selected from the group consisting of sodium N-stearoyl-N-methyltaurine and sodium N-stearoyl-L-glutamate.
<42> An oil-in-water emulsion composition comprising the following components (A), (B), (C), and (D):
   (A) a ceramide,
   (B) an oil agent in a liquid state at 25°C,
   (C) a nonionic surfactant having HLB of less than 10, and
   (D) water, wherein
      an average particle size of emulsion particles is from 0.05 to 0.3 µm,
      the component (B) comprises
         (B1) a hydrocarbon oil, and
         (B2) 0.05 to 3.5% by mass of a polar oil, and
      a mass ratio of the component (A) to the component (B), (A)/(B), is from 0.05 to 1.

### Examples

### Examples 1 to 19 and Comparative Examples 1 and 2

Each oil-in-water emulsion composition was produced in accordance with the composition shown in Tables 1 to 4. Emulsion particle sizes were measured immediately after production. The composition was evaluated for stability (45°C, 2 weeks), moist feeling and smoothness in the skin after being applied to the skin with fingers, and moist feeling and smoothness in the skin after being applied to the skin with impregnated cotton. The results are also shown in Tables 1 to 4.

The oil-in-water emulsion compositions of Examples 1 to 19 can also be used as makeup removers.

### (Production method)

(1) The components (A) to (C), a portion of the component (D) (0.94% by mass of the whole component (D)), the component (E), stearoyl methyltaurine, and 1,3-butylene glycol were uniformly mixed (150 rpm) and dissolved at 80°C to prepare an oil phase.
(2) Water (99.06% by mass of the whole component (D)) in the rest of the component (D) and glycerin were uniformly mixed and dissolved at 80°C to prepare an aqueous phase.
(3) The oil phase obtained in (1) was added at 80°C to the aqueous phase obtained in (2), and mixed (300 rpm), and the mixture was stirred for 10 minutes and then allowed to cool to room temperature (25°C) to obtain an oil-in-water emulsion composition.

### (Evaluation method)

### (1) Particle size:

Immediately after production, each oil-in-water emulsion composition was diluted 20-fold, and the Z-average particle size of emulsion particles was measured at 25°C by using Zetasizer Nano Z manufactured by Malvern Panalytical Ltd. Here, the Z-average particle size was set to an average value of three measurement values. Here, a cell of 10 × 10 × 45 mm in length manufactured by Sarstedt K.K. was subject to the measurement.

### (2) Presence or absence of large particles:

In the measurement results of (1), the proportion of 1 µm or larger particles was evaluated from a particle size distribution in accordance with the following criteria.
3: Less than 1%.
2: 1% or more and less than 5%.
1: 5% or more.

### (3) Stability (45°C, 2 weeks):

20 mL of each oil-in-water emulsion composition was placed in a 28 mL transparent glass container (Mighty Vial No. 6, manufactured by Maruemu Corp.), which was then hermetically sealed and preserved at 45°C for 2 weeks. Then, its appearance was evaluated in accordance with the following criteria.
5: A uniform single layer, no separation observed.
4: Although an upper layer has wispy cloudiness, visually confirmable cloudiness is no longer present by mild shaking, resulting in no practical problem.
3: An upper layer has separation of less than 1 mm, which can be visually confirmed even after mild shaking.
2: An upper layer has separation of 1 mm or more and less than 5 mm, which can be visually confirmed even after mild shaking.
1: An upper layer has separation of 5 mm or more.

### (4) Moist feeling and smoothness in skin after application (applied to skin with fingers):

Three specialized evaluators applied 0.5 mL of each oil-in-water emulsion composition to an area of 4 × 10 cm on the inner side of the forearm and spread the composition over the skin for approximately 20 seconds by three fingers (long fingers). Then, moist feeling and smoothness in the skin were evaluated in accordance with the criteria given below. The results were indicated by a total score from the three specialized evaluators.

### (4-1) Moist feeling:

5: Evidently moist.
4: Moist.
3: Slightly moist.
2: Barely moist.
1: Evidently not moist.

### (4-2) Smoothness:

5: Evidently smooth.
4: Smooth.
3: Slightly smooth.
2: Barely smooth.
1: Evidently not smooth.

### (5) Moist feeling and smoothness in skin after application (applied to skin with impregnated cotton)

Three specialized evaluators put 2 mL of each oil-in-water emulsion composition onto cotton (3.5 × 6.5 cm, Beauty Works Daily Cotton, manufactured by Kanebo Cosmetics Inc.) and wiped an area of 4 × 10 cm on the inner side of the forearm. Then, moist feeling and smoothness in the skin were evaluated in accordance with the same criteria as in (4-1) and (4-2). The results were indicated by a total score from the three specialized evaluators.

**[Table 1]**

| | | Component (% by mass) | Example | Example | Example | Example | Example | Example | Example | Comparative Example | Comparative Example |
|---|---|---|---|---|---|---|---|---|---|---|---|
| | | | 1 | 2 | 3 | 4 | 5 | 6 | 7 | 1 | 2 |
| (A) | | N-(2-Hydroxy-3-hexadecyloxypropyl)-N-2-hydroxyethylhexadecanamide *1 | 1 | 0.5 | 1.5 | 1 | 1 | 1 | 1 | 1 | 1 |
| (B) | (B1) | Hydrogenated polyisobutene *2 | 2 | 1 | 2 | 1 | 2 | 2 | 2 | | 2 |
| | (B2) | Neopentyl glycol dicaprate *3 | 2 | 1 | 2 | 1 | 2 | 2 | 2 | | 2 |
| | | Dimethylpolysiloxane *4 | 2 | 1 | 2 | 2 | 2 | 2 | 2 | | 2 |
| (C) | | Sorbitan stearate (HLB: 4.7) *5 | 0.8 | 0.8 | 0.8 | 0.8 | 0.6 | 1.2 | 1.6 | 0.8 | |
| (E) | | Polyoxyethylene (20EO) sorbitan stearate (HLB: 14.9) *6 | 1.6 | 1.6 | 1.6 | 1.6 | 1.6 | 1.6 | 1.6 | 1.6 | 2.4 |
| | | Stearoyl methyltaurine *7 | 0.2 | 0.2 | 0.2 | 0.2 | 0.2 | 0.2 | 0.2 | 0.2 | 0.2 |
| | | 1,3-Butylene glycol *8 | 2 | 2 | 2 | 2 | 2 | 2 | 2 | 2 | 2 |
| | | Glycerin | 3 | 3 | 3 | 3 | 3 | 3 | 3 | 3 | 3 |
| (D) | | Water | 85.4 | 88.9 | 84.9 | 87.4 | 85.6 | 85 | 84.6 | 91.4 | 85.4 |
| Total | | | 100 | 100 | 100 | 100 | 100 | 100 | 100 | 100 | 100 |
| (A) total amount | | | 1 | 0.5 | 1.5 | 1 | 1 | 1 | 1 | 1 | 1 |
| (B) total amount | | | 6 | 3 | 6 | 4 | 6 | 6 | 6 | 0 | 6 |
| (C) total amount | | | 0.8 | 0.8 | 0.8 | 0.8 | 0.6 | 1.2 | 1.6 | 0.8 | 0 |
| (E) total amount | | | 1.6 | 1.6 | 1.6 | 1.6 | 1.6 | 1.6 | 1.6 | 1.6 | 2.4 |
| Ionic surfactant | | | 0.2 | 0.2 | 0.2 | 0.2 | 0.2 | 0.2 | 0.2 | 0.2 | 0.2 |
| Polyhydric alcohol | | | 5 | 5 | 5 | 5 | 5 | 5 | 5 | 5 | 5 |
| Dihydric alcohol | | | 2 | 2 | 2 | 2 | 2 | 2 | 2 | 2 | 2 |
| (A)/(B) | | | 0.17 | 0.17 | 0.25 | 0.25 | 0.17 | 0.17 | 0.17 | - | 0.17 |
| (A)/(C) | | | 1.25 | 0.63 | 1.88 | 1.25 | 1.67 | 0.83 | 0.63 | 1.25 | - |
| (B)/(C) | | | 7.5 | 3.75 | 7.5 | 5 | 10 | 5 | 3.75 | - | - |
| (B1)/(B2) | | | 1 | 1 | 1 | 1 | 1 | 1 | 1 | - | 1 |
| Particle size (µm) immediately after | | | 0.13 | 0.08 | 0.17 | 0.09 | 0.11 | 0.1 | 0.18 | 0.18 | 0.51 |
| Presence or absence of large particle | | | 3 | 3 | 3 | 3 | 3 | 3 | 3 | 1 | 1 |
| Stability (45°C, 2 wk) | | | 5 | 5 | 5 | 5 | 5 | 5 | 5 | 1 | 1 |
| Applied to skin with fingers: Moist feeling in skin after application | | | 15 | 13 | 15 | 13 | 15 | 15 | 15 | 9 | 15 |
| Smoothness in skin after application | | | 15 | 11 | 15 | 11 | 15 | 15 | 15 | 5 | 15 |
| Applied to skin with impregnated cotton: Moist feeling in skin after application | | | 15 | 13 | 15 | 12 | 15 | 15 | 15 | 9 | 15 |
| Smoothness in skin after application | | | 15 | 12 | 15 | 12 | 15 | 15 | 15 | 5 | 15 |

**[Table 2]**

| | | Component (% by mass) | Example | Example | Example |
|---|---|---|---|---|---|
| | | | 1 | 8 | 9 |
| (A) | | N-(2-Hydroxy-3-hexadecyloxypropyl)-N-2-hydroxyethylhexadecanamide *1 | 1 | 1 | 1 |
| (B) | (B1) | Hydrogenated polyisobutene *2 | 2 | 2 | 2 |
| | (B2) | Neopentyl glycol dicaprate *3 | 2 | | 2 |
| | | Isononyl isononanoate | | 2 | |
| | | Dimethylpolysiloxane *4 | 2 | 2 | 2 |
| (C) | | Sorbitan stearate (HLB: 4.7) *5 | 0.8 | 0.8 | |
| | | Monoglyceride stearate (HLB: 3.5) | | | 0.8 |
| (E) | | Polyoxyethylene (20EO) sorbitan stearate (HLB: 14.9) *6 | 1.6 | 1.6 | 1.6 |
| | | Stearoyl methyltaurine *7 | 0.2 | 0.2 | 0.2 |
| | | 1,3-Butylene glycol *8 | 2 | 2 | 2 |
| | | Glycerin | 3 | 3 | 3 |
| (D) | | Water | 85.4 | 85.4 | 85.4 |
| Total | | | 100 | 100 | 100 |
| (A) total amount | | | 1 | 1 | 1 |
| (B) total amount | | | 6 | 6 | 6 |
| (C) total amount | | | 0.8 | 0.8 | 0.8 |
| (E) total amount | | | 1.6 | 1.6 | 1.6 |
| Ionic surfactant | | | 0.2 | 0.2 | 0.2 |
| Polyhydric alcohol | | | 5 | 5 | 5 |
| Dihydric alcohol | | | 2 | 2 | 2 |
| (A)/(B) | | | 0.17 | 0.17 | 0.17 |
| (A)/(C) | | | 1.25 | 1.25 | 1.25 |
| (B)/(C) | | | 7.5 | 7.5 | 7.5 |
| (B1)/(B2) | | | 1 | 1 | 1 |
| Particle size (µm) immediately after | | | 0.13 | 0.10 | 0.11 |
| Presence or absence of large particle | | | 3 | 3 | 3 |
| Stability (45°C, 2 wk) | | | 5 | 5 | 5 |
| Applied to skin with fingers: Moist feeling in skin after application | | | 15 | 14 | 15 |
| Smoothness in skin after application | | | 15 | 13 | 15 |
| Applied to skin with impregnated cotton: Moist feeling in skin after application | | | 15 | 14 | 15 |
| Smoothness in skin after application | | | 15 | 13 | 15 |

**[Table 3]**

| | | Component (% by mass) | Example | Example | Example | Example | Example | Example |
|---|---|---|---|---|---|---|---|---|
| | | | 10 | 11 | 12 | 13 | 14 | 15 |
| (A) | | N-(2-Hydroxy-3-hexadecyloxypropyl)-N-2-hvdroxvethylhexadecanamide *1 | 1 | 1 | 1.5 | 0.5 | 1 | 1 |
| (B) | (B1) | Hvdroaenated polyisobutene *2 | 2 | 2 | 2 | 2 | 2 | 2 |
| | (B2) | Neopentyl glycol dicaprate *3 | | 0.5 | 2 | 2 | 2 | 2 |
| | | Isotridecyl isononanoate *9 | 3 | | | | | |
| | | Dimethylpolysiloxane *4 | 2 | 2 | 2 | 2 | 2 | 2 |
| (C) | | Sorbitan stearate (HLB: 4.7) *5 | 0.8 | 0.8 | 0.3 | 2.4 | 0.8 | 0.8 |
| (E) | | Polyoxyethylene (20EO) sorbitan stearate (HLB: 14.9) *6 | 1.6 | 1.6 | 1.6 | 2.4 | 1.6 | 1.6 |
| | | Stearoyl methyltaurine *7 | 0.2 | 0.2 | 0.2 | 0.2 | 0.5 | 1 |
| | | 1,3-Butylene glycol *8 | 2 | 2 | 2 | 2 | 2 | 2 |
| | | Glycerin | 3 | 3 | 3 | 3 | 3 | 3 |
| (D) | | Water | Balance | Balance | Balance | Balance | Balance | Balance |
| Total | | | 100 | 100 | 100 | 100 | 100 | 100 |
| (A) total amount | | | 1 | 1 | 1.5 | 0.5 | 1 | 1 |
| (B) total amount | | | 7 | 4.5 | 6 | 6 | 6 | 6 |
| (C) total amount | | | 0.8 | 0.8 | 0.3 | 2.4 | 0.8 | 0.8 |
| (E) total amount | | | 1.6 | 1.6 | 1.6 | 2.4 | 1.6 | 1.6 |
| Ionic surfactant | | | 0.2 | 0.2 | 0.2 | 0.2 | 0.5 | 1 |
| Polyhydric alcohol | | | 2 | 2 | 2 | 2 | 2 | 2 |
| (A)/(B) | | | 0.14 | 0.22 | 0.25 | 0.08 | 0.17 | 0.17 |
| (A)/(C) | | | 1.25 | 1.25 | 5 | 0.21 | 1.25 | 1.25 |
| (B)/(C) | | | 8.75 | 5.63 | 20 | 2.5 | 7.5 | 7.5 |
| (B1)/(B2) | | | 0.67 | 4 | 1 | 1 | 1 | 1 |
| Particle size (µm) immediately after | | | 0.12 | 0.08 | 0.16 | 0.16 | 0.08 | 0.07 |
| Presence or absence of large particle | | | 3 | 3 | 3 | 3 | 3 | 3 |
| Stability (45°C, 2 wk) | | | 3 | 4 | 5 | 3 | 5 | 5 |
| Applied to skin with fingers: Moist feeling in skin after application | | | 14 | 13 | 15 | 15 | 15 | 15 |
| Smoothness in skin after application | | | 15 | 15 | 15 | 15 | 15 | 12 |
| Applied to skin with impregnated cotton: Moist feeling in skin after application | | | 14 | 13 | 15 | 15 | 15 | 15 |
| Smoothness in skin after application | | | 15 | 15 | 15 | 15 | 15 | 12 |

**[Table 4]**

| | | Component (% by mass) | Example | Example | Example | Example |
|---|---|---|---|---|---|---|
| | | | 16 | 17 | 18 | 19 |
| (A) | | N-(2-Hydroxy-3-hexadecyloxypropyl)-N-2-hvdroxvethylhexadecanamide *1 | 1 | 1 | 0.6 | |
| | | Ceramide 2 *10 | | | 0.2 | 0.5 |
| | | Ceramide 3 *11 | | | 0.2 | 0.5 |
| (B) | (B1) | Hvdroaenated polyisobutene *2 | 2 | | 2 | 2 |
| | | Squalane *12 | | 2 | | |
| | (B2) | Neopentyl glycol dicaprate *3 | 2 | | 2 | 2 |
| | | Isononyl isononanoate *13 | | | | |
| | | Isostearyl isostearate *14 | | 0.5 | | |
| | | Jojoba oil *15 | | 0.5 | | |
| | | Olive oil *16 | | 0.5 | | |
| | | Glvcervl tri(caprate-caprylate) *17 | | 0.5 | | |
| | | Dimethylpolysiloxane *4 | 2 | 2 | 2 | 2 |
| (C) | | Sorbitan stearate (HLB: 4.7) *5 | | | | |
| | | Monoglyceride stearate (HLB: 3.5) *18 | 1.2 | 0.8 | 0.8 | 0.8 |
| (E) | | Polvoxvethylene (20EO) sorbitan stearate (HLB: 14.9) *6 | 2.4 | 2.4 | 1.6 | 1.6 |
| | | Stearoyl methyltaurine *7 | | 0.5 | 0.5 | 0.2 |
| | | Na stearovl glutamate *19 | 0.5 | | | |
| | | 1,3-Butylene glycol *8 | 2 | 2 | 2 | 2 |
| | | Glycerin | 3 | 3 | 3 | 3 |
| (D) | | Water | Balance | Balance | Balance | Balance |
| Total | | | 100 | 100 | 100 | 100 |
| (A) total amount | | | 1 | 1 | 1 | 1 |
| (B) total amount | | | 6 | 6 | 6 | 6 |
| (C) total amount | | | 1.2 | 0.8 | 0.8 | 0.8 |
| (E) total amount | | | 2.4 | 2.4 | 1.6 | 1.6 |
| Ionic surfactant | | | 0.5 | 0.5 | 0.5 | 0.2 |
| Polyhydric alcohol | | | 5 | 5 | 5 | 5 |
| (A)/(B) | | | 0.17 | 0.17 | 0.17 | 0.17 |
| (A)/(C) | | | 0.83 | 1.25 | 1.25 | 1.25 |
| (B)/(C) | | | 5 | 7.5 | 7.5 | 7.5 |
| (B1)/(B2) | | | 1 | 1 | 1 | 1 |
| Particle size (µm) immediately after | | | 0.07 | 0.06 | 0.06 | 0.1 |
| Presence or absence of large particle | | | 3 | 3 | 3 | 3 |
| Stability (45°C, 2 wk) | | | 5 | 5 | 5 | 5 |
| Applied to skin with fingers: Moist feeling in skin after application | | | 15 | 15 | 15 | 15 |
| Smoothness in skin after application | | | 10 | 10 | 15 | 15 |
| Applied to skin with impregnated cotton: Moist feeling in skin after application | | | 15 | 15 | 15 | 15 |
| Smoothness in skin after application | | | 10 | 10 | 15 | 15 |

| | | | | | | |
|---|---|---|---|---|---|---|
| *1: N-(Hexadecyloxyhydroxypropyl)-N-hydroxyethylhexadecanamide; Sphingolipid E (manufactured by Kao Corp.) *2: Hydrogenated polyisobutene; PARLEAM EX (manufactured by NOF Corp.) *3: Neopentyl glycol dicaprate; ESTEMOL N-01 (manufactured by The Nisshin OilliO Group, Ltd.) *4: Dimethylpolysiloxane; Silicone 2CS (manufactured by Shin-Etsu Chemical Co., Ltd.) *5: Sorbitan stearate (HLB: 4.7); RHEODOL SP-S10V (manufactured by Kao Corp.) *6: Polyoxyethylene (20EO) sorbitan stearate (HLB: 14.9); RHEODOL TW-S120V (manufactured by Kao Corp.) *7: Stearoyl methyltaurine; NIKKOL SMT (manufactured by Nippon Surfactant Industries Co., Ltd.) *8: 1,3-Butylene glycol; 1,3-Butylene Glycol (manufactured by KH Neochem Co., Ltd.) *9: Isotridecyl isononanoate; SALACOS 913 (manufactured by The Nisshin OilliO Group, Ltd.) *10: Ceramide 2; CERAMIDE TIC-001 (manufactured by Takasago International Corp.) *11: Ceramide 3; CERAMIDE III (manufactured by Evonik industries AG) *12: Squalane; NIKKOL Sugar Squalane (manufactured by Nikko Chemicals Co., Ltd.) *13: Isononyl isononanoate; SALACOS 99 (manufactured by The Nisshin OilliO Group, Ltd.) *14: Isostearyl isostearate; ISIS (manufactured by Kokyu Alcohol Kogyo Co., Ltd.) *15: Jojoba oil; Purified Jojoba Oil (manufactured by Koei Kogyo Co., Ltd.) *16: Olive oil; CROPURE OL (manufactured by Croda Japan K.K.) *17: Glyceryl tri(caprylate-caprate); COCONARD MT (manufactured by Kao Corp.) *18: Monoglyceride stearate (HLB: 3.5); RHEODOL MS-60 (manufactured by Kao Corp.) *19: Na stearoyl glutamate; AMISOFT HS-11P (manufactured by Ajinomoto Co., Inc.) | | | | | | |

### Formulation Examples 1 to 3 (makeup remover)

Oil-in-water emulsion cosmetics (makeup removers) shown in Table 5 were produced in the same manner as in Examples 1 to 19. These cosmetics are excellent in preservation stability and also excellent in makeup removing effect and can impart smoothness and excellent moist feeling in the skin after application because an oil phase containing a ceramide is stably emulsified and dispersed as fine particles.

**[Table 5]**

| | | Component (% by mass) | Formulation Example 1 | Formulation Example 2 | Formulation Example 3 |
|---|---|---|---|---|---|
| (A) | | N-(2-Hydroxy-3-hexadecyloxypropyl)-N-2-hydroxyethylhexadecanamide *1 | 1 | 1 | 0.5 |
| | | Cetyl alcohol | 0.6 | 0.6 | 1.2 |
| | | Stearyl alcohol | 0.4 | 0.4 | 0.8 |
| | | Cholesterol | 0.4 | 0.4 | 0.2 |
| | | Cholesteryl isostearate | 0.3 | 0.3 | 0.2 |
| (B) | (B1) | Hydrogenated polyisobutene *2 | 2 | 2 | 2 |
| | (B2) | Neopentyl glycol dicaprate *3 | 2 | 2 | 2 |
| | | Dimethylpolysiloxane *4 | 2 | 2 | 2 |
| (C) | | Sorbitan stearate (HLB: 4.7) *5 | 0.8 | 0.8 | 0.8 |
| (E) | | Polyoxyethylene (20EO) sorbitan stearate (HLB: 14.9) *6 | 1.6 | 1.6 | 1.6 |
| | | Stearoyl methyltaurine *7 | 0.4 | 0.4 | 0.4 |
| | | 1,3-Butylene glycol *8 | 5 | 5 | 5 |
| | | Dipropylene glycol | 3 | 3 | 5 |
| | | Glycerin | 3 | 3 | 5 |
| | | Carbomer | | 0.2 | 0.2 |
| | | Arginine | | 0.05 | 0.05 |
| | | Dipotassium glycyrrhizinate | 0.1 | 0.1 | 0.1 |
| | | Eucalyptus extract | 0.5 | 0.5 | 0.5 |
| | | Methylparaben | 0.2 | 0.2 | 0.2 |
| (D) | | Water | 76.7 | 76.45 | 72.25 |
| Total | | | 100 | 100 | 100 |
| (A) total amount | | | 1 | 1 | 0.5 |
| (B) total amount | | | 6 | 6 | 6 |
| (C) total amount | | | 0.8 | 0.8 | 0.8 |
| (E) total amount | | | 1.6 | 1.6 | 1.6 |
| Ionic surfactant | | | 0.4 | 0.4 | 0.4 |
| Polyhydric alcohol | | | 11 | 11 | 15 |
| Dihydric alcohol | | | 8 | 8 | 10 |
| (A)/(B) | | | 0.17 | 0.17 | 0.08 |
| (A)/(C) | | | 1.25 | 1.25 | 0.63 |
| (B)/(C) | | | 7.5 | 7.5 | 7.5 |
| (B1)/(B2) | | | 1 | 1 | 1 |
| Particle size (µm) | | | 0.17 | 0.11 | 0.19 |

## Claims

1. An oil-in-water emulsion composition comprising the following components (A), (B), (C), and (D) :
(A) a ceramide,
(B) an oil agent in a liquid state at 25°C,
(C) a nonionic surfactant having HLB of less than 10, and
(D) water, wherein
a Z-average particle size of emulsion particles is from 0.05 to 0.3 µm.

2. The oil-in-water emulsion composition according to claim 1, further comprising an anionic surfactant.

3. The oil-in-water emulsion composition according to claim 1 or 2, wherein a mass ratio of the component (A) to the component (C), (A)/(C), is from 0.1 to 5.

4. The oil-in-water emulsion composition according to any one of claims 1 to 3, wherein a mass ratio of the component (B) to the component (C), (B)/(C), is from 1 to 20.

5. The oil-in-water emulsion composition according to any one of claims 1 to 4, wherein the component (B) comprises (B1) a hydrocarbon oil and (B2) a polar oil.

6. The oil-in-water emulsion composition according to any one of claims 1 to 5, further comprising (E) a nonionic surfactant having HLB of 10 or more.

7. An oil-in-water emulsion composition comprising the following components (A), (B), (C), and (D) :
(A) a ceramide,
(B) an oil agent in a liquid state at 25°C,
(C) a nonionic surfactant having HLB of less than 10, and
(D) water, and
an anionic surfactant, wherein
a Z-average particle size of emulsion particles is from 0.05 to 0.3 µm.

8. The oil-in-water emulsion composition according to any one of claims 2 to 7, wherein the anionic surfactant comprises one or more members selected from the group consisting of N-alkyloyl methyltaurine having 12 to 22 carbon atoms or a salt thereof, and N-C12 to C22 acyl glutamic acid or a salt thereof.

9. The oil-in-water emulsion composition according to any one of claims 1 to 8, for use as a makeup remover.

10. A method for producing the oil-in-water emulsion composition according to claim 1, comprising: a step 1 of obtaining an oil phase containing the component (A), the component (B), and the component (C); and a step 2 of mixing the oil phase obtained in the step 1 with an aqueous phase containing the component (D).
